Europäisches Patentamt

European Patent Office

Office européen des brevets

(11) Publication number: **0 105 618**
**B1**

(12) **EUROPEAN PATENT SPECIFICATION**

(45) Date of publication of patent specification: **25.10.89**

(21) Application number: **83305110.5**

(22) Date of filing: **02.09.83**

(51) Int. Cl.⁴: **G 01 N 23/04,** G 21 K 1/02, A 61 B 6/00

(54) X-Ray imaging system having radiation scatter compensation and method.

(30) Priority: **07.09.82 US 415333**

(43) Date of publication of application:
**18.04.84 Bulletin 84/16**

(45) Publication of the grant of the patent:
**25.10.89 Bulletin 89/43**

(84) Designated Contracting States:
**DE FR GB NL**

(56) References cited:
**EP-A-0 028 431**
**EP-A-0 093 649**
**DE-A-2 452 166**
**GB-A-2 021 896**
**US-A-4 081 681**

**APPLIED OPTICS, vol. 15, no. 3, March 1976, pages 648-655, New York, US; G. KOWALSKI: "Suppression of scattered radiation in radiography and improvement of resolution by spatially modulated intensity"**

(73) Proprietor: **THE BOARD OF TRUSTEES OF THE LELAND STANFORD JUNIOR UNIVERSITY**
**Office of Technology and Licensing 105 Encina Hall Stanford University**
**Stanford California 94305 (US)**

(72) Inventor: **Macovski, Albert (NMI)**
**2505 Alpine Road**
**Menlo Park California 94025 (US)**

(74) Representative: **Allard, Susan Joyce et al**
**BOULT, WADE & TENNANT 27 Furnival Street**
**London EC4A 1PQ (GB)**

Courier Press, Leamington Spa, England.

## Description

This invention relates generally to X-ray imaging systems, and more particularly the invention relates to a method of compensating such systems for radiation scatter and to the resulting system.

X-ray imaging systems are used for medical diagnostic purposes and for non-destructively inspecting the internal composition of various structures. A patient or specimen is irradiated by an X-ray beam and the elements of the specimen attenuate the radiation. A radiation detector detects the attenuated radiation and generates an electrical signal indicative thereof. The electrical signal is then used to control a suitable monitor such as a TV receiver.

Radiation scatter is a significant source of error in all X-ray imaging systems employing area detectors. Fluoroscopic systems have an additional source of error due to optical scatter known as veiling glare. Energy selective imaging systems, such as dual energy systems, are particularly sensitive to scatter because of the subsequent processing.

GB—A—2021896 discloses a computer assisted tomography apparatus which compensates for radiation scatter. The apparatus described comprises a detector having a first region in the plane of examination shielded from direct radiation from the source and a further region out of the said plane and sensitive to scattered radiation. An image is obtained when the first region is unshielded. Correction of this image is obtained by subtracting the scattered radiation signal derived from the further region.

In addition, US—A—4081681 discloses compensating for radiation scatter in a tomographic apparatus by estimating the distribution of absorption co-efficients and deriving an indication of the appropriate scatter compensation therefrom.

Reference may also be made to EP—A—0093649 (published on 09.11.83) which discloses a compensation method similar to that of the present invention.

Accordingly, an object of the present invention is a method of compensating for scatter in an X-ray imaging system.

Another object of the invention is an X-ray imaging system which compensates for radiation scatter.

A feature of the invention is the measurement of scattered radiation through use of an opaque shield placed in an imaging system whereby scattered radiation only is received by the shielded areas of the detector. The scattered radiation for the entire display can then be interpolated from the measured scattered radiation in the shield areas of the detector.

Advantageously, the use of a shield for determining scattered radiation can be limited in area whereby scattered radiation is determined while the specimen is being imaged.

In one embodiment of an X-ray imaging system in accordance with the invention which utilizes a collimator for defining the X-ray beam pattern, a measurement of scattered radiation can be obtained by detecting the radiation in the area of the detector shield by the collimator.

The invention and objects and features thereof will be more readily apparent from the following detailed description and appended claims when taken with the drawing, in which:

Figure 1 is a functional block diagram of an X-ray imaging system having scatter compensation in accordance with the invention.

Figure 2 is an alternative block diagram of an X-ray imaging system with scatter compensation in accordance with the invention.

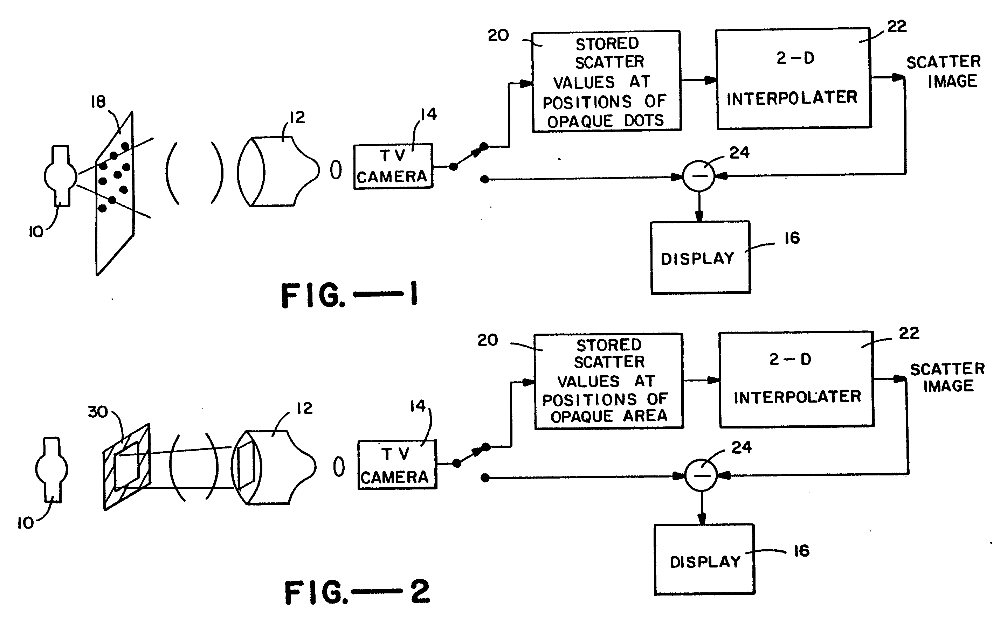

Referring now to the drawing, Figure 1 is a functional block diagram of an X-ray imaging system such as fluoroscopic system in which X-radiation from a source 10 is directed through a specimen (not shown) to an image intensifier 12, such as scintillators, which receives and intensifies the attenuated radiation passing through the specimen. Light from the intensifier 21 is received by TV camera 14 which generates a video signal. The intensifier and camera collectively form a radiation detector. The video signal from camera 14 is then applied to a display monitor 16 for viewing the internal structure of the specimen as determined by the attenuated radiation passing therethrough.

As above noted, radiation received by the image intensifier 12 includes a significant amount of scattered radiation which can cause a significant error in the displayed image. In accordance with the invention a measure of the scattered radiation is determined, and the video signal is then compensated by substracting the measured scattered radiation. In the embodiment of Figure 1 this is accomplished by providing an array 18 of X-ray opaque dots, for example, in the path between the X-ray source 10 and the image intensifier 12. Radiation received by the intensifier 12 aligned with the array of X-ray opaque dots will be solely scattered radiation since the direct passage of radiation is blocked by the opaque dots. Thus, the video signal of the detected radiation at the surfaces corresponding to the dot pattern is then stored in a memory 20. A two dimensional interpolator 22 then interpolates the scattered radiation across the entire surface of the detector 12 based on the geometric pattern of the X-ray opaque dots and the radiation measured at the corresponding positions on the surface of detector 12. The interpolated attenuation for the display is then subtracted at 24 from the video signal from camera 14 to provide a control signal for display 16 which is relatively free of error due to scatter.

The system as described with reference to Figure 1 makes the basic assumption that the scatter is a low spatial frequency phenomena. Therefore, a relatively coarse set of samples of the scattered field will suffice to reconstruct the complete scatter distribution. The detected intensities at the positions corresponding to the opaque dots are recorded and used in the two dimensional interpolation arrangement to estimate the amount of veiling glare and scatter throughout the image plane. The estimated values are then subtracted from the measured intensities during the study.

The scatter calibration can be done with a fluoroscopic system by recording the values from the camera output corresponding to the position of the X-ray opaque dots. In other X-ray imaging systems, such as film systems, an array of detectors can be used in positions corresponding to the array of opaque dots to record the scatter. Alternatively, the film can be optically scanned with the values corresponding to the position of the opaque dots recorded and stored.

To provide a higher degree of sampling of the scatter field, an increased number of opaque dots can be used to provide greater accuracy. However, a relatively large number of dots will begin to substantially affect the amount of scatter produced. Accordingly, a predetermined weighting factor can be determined experimentally and applied to the measurements to account for the reduction of scatter.

In the case of a multiple energy system, stored scatter values are provided at each energy since the scatter is a function of energy. In some cases the scatter distribution at the different energies may differ not only by a known constant weighting factor, therefore the scatter at a given energy level can be derived from a measurement at a different energy level.

Figure 2 is a functional block diagram of an alternative embodiment similar to that of Figure 1 but including a rectangular collimator 30 for limiting the field of the X-ray beam passing through a specimen. In this embodiment scattered radiation can be determined by measuring the radiation on the surface of the detector 12 corresponding to the surface of the collimator 30. The advantage of this embodiment is that the X-radiation passing through the specimen is not interrupted. However, the spatial distribution of the scatter may not be as accurate as that determined through use of X-ray opaque dots as in Figure 1. In an alternative embodiment to that shown in Figure 2 a limited number of opaque dots (such as used in Figure 1) can be added to the system of Figure 2 to more accurately estimate the spatial distribution of the scattered radiation.

The estimation and subtraction can be a one step or a two step operation. In the one step operation the image of opaque dots are left in the final image since they are used to estimate the scatter. If these dots are found to be undesirable, a second image is taken with the array of dots 18 removed. As shown in Figure 1, the switch is placed on position 2, with the scatter image subtracted from the camera signal, which is now free of dots. In this two step system the dots are removed at the price of increased radiation.

The number of dots required to accurately represent the scatter depends on the smoothness of the scattered radiation. This smoothness can be enhanced by separating the specimen from the detector, thus allowing the scattered radiation to spread and become more uniform. This will minimize the number of required dots so that they will become less objectionable when the one step system is used.

Through use of a scatter measurement and compensation system in accordance with the invention more accurate images can be developed of a specimen under examination.

**Claims for the Contracting States: FR, GB**

1. An X-ray imaging system comprising:
an X-ray source (10),
an X-ray detector means (12) positioned to receive and detect X-radiation from the source and generate an electrical signal indication thereof,
radiation shield means (10) positioned between the source (10) and the detector means (12) to prevent radiation from passing directly to shielded areas of the detector means, characterised in having interpolation means (22) for receiving electrical signals from the detector means (12) indicative of scattered radiation received by the shielded areas of the detector means (12) and for interpolating therefrom a scattered radiation signal for all of the detector means (12), and
means (24) for subtracting the interpolated scattered radiation signal from the electrical signal generated by the detector means (12).

2. An X-ray imaging system as claimed in Claim 1 wherein the shield means (18) comprises a plurality of radiation opaque bodies positioned between the source (10) and the detector means (12) and spatially distributed for facilitating interpolation of a scattered radiation signal for all the area of the detector means (12).

3. An X-ray imaging system as claimed in Claim 1 or Claim 2 wherein the shield means (18) comprises a beam collimator (30) for limiting the geometry of an X-ray beam received by the detector means (12) directly from the source (10).

4. An X-ray imaging system as claimed in any one of the preceding claims wherein the X-ray detector means (12) comprises an image intensifier and a TV camera (14).

5. A method of compensating for scatter radiation in an X-ray imaging system as claimed in Claim 1 comprising the steps of:
positioning radiation shield means (18) between the radiation source (10) and the radiation detector means (12),
measuring radiation received by the radiation detector means (12) at the shielded areas,
interpolating (22) scattered radiation for all of the detector means (12) from the measured radiation at the shielded areas,
measuring radiation received by the radiation means (12) at all unshielded areas, and
subtracting (24) the interpolated scattered radiation from the measured X-radiation.

6. A method as claimed in Claim 5 wherein the steps of measuring radiation are concurrent.

7. A method as claimed in Claim 5 including the step of removing the radiation shield (18) and measuring the radiation received at all areas.

**Claims for the Contracting States: DE, NL**

1. An X-ray imaging system comprising:
an X-ray source (10),
an X-ray detector means (12) positioned to receive and detect X-radiation from the source and generate an electrical signal indication thereof,
radiation shield means (18) positioned between the source (10) and the detector means (12) to prevent radiation from passing directly to shielded areas of the detector means, characterised in having interpolation means (22) for receiving electrical signals from the detector means (12) indicative of scattered radiation received by the shielded areas of the detector means (12) and for interpolating therefrom a scattered radiation signal for all of the detector means (12), and
means (24) for subtracting the interpolated scattered radiation signal from the electrical signal generated by the detector means (12).

2. An X-ray imaging system as claimed in Claim 1 wherein the shield means (18) comprises a plurality of radiation opaque bodies positioned between the source (10) and the detector means (12) and spatially distributed for facilitating interpolation of a scattered radiation signal for all the area of the detector means (12).

3. An X-ray imaging system as claimed in Claim 1 or Claim 2 wherein the shield means (18) comprises a beam collimator (30) for limiting the geometry of an X-ray beam received by the detector means (12) directly from the source (10).

4. An X-ray imaging system as claimed in any one of the preceding claims wherein the X-ray detector means (12) comprises an image intensifier and a TV camera (14).

**Patentansprüche fur die Vertragsstaaten: FR, GB**

1. Röntgenstrahl-Abbildungssystem mit:
einer Röntgenstrahlenquelle (10),
einer Röntgenstrahl-Detektoreinrichtung (12), die so angeordnet ist, daß sie von der Quelle Röntgenstrahlung empfängt und erfaßt und ein die Strahlung anzeigendes elektrisches Signal erzeugt,
eine Strahlungs-Abschirmeinrichtung (18), die zwischen der Quelle (10) und der Detektoreinrichtung (12) angeordnet ist und verhindert, daß Strahlung direkt zu abgeschirmten Bereichen der Detektoreinrichtung gelangt, gekennzeichnet durch eine Interpoliereinrichtung (22) zum Empfang elektrischer Signale von der Detektoreinrichtung (12), die von den abgeschirmten Bereichen der Detektoreinrichtung (12) empfangene Streustrahlung anzeigt und daraus ein Streustrahlungs-Signal für alle Detektoreinrichtungen (12) interpoliert, und eine Einrichtung (24) zum Subtrahieren des interpolierten Streustrahlungs-Signals von dem von der Detektoreinrichtung (12) erzeugten elektrischen Signal.

2. Röntgenstrahl-Abbildungssystem nach Anspruch 1, wobei die Abschirmeinrichtung (18) mehrere strahlungsundurchlässige Körper aufweist, die zwischen der Quelle (10) und der Detektoreinrichtung (12) angeordnet und räumlich verteilt sind, um die Interpolation eines Streustrahlungs-Signals für die gesamte Fläche der Detektoreinrichtung (12) zu erleichtern.

3. Röntgenstrahl-Abbildungssystem nach Anspruch 1 oder 2, wobei die Abschirmeinrichtung (18) einen Strahlkollimator (30) aufweist, um die Geometrie eines von der Detektoreinrichtung (12) direkt von der Quelle (10) empfangenen Röntgenstrahls zu begrenzen.

4. Röntgenstrahl-Abbildungssystems nach einem der vorstehenden Ansprüche, wobei die Röntgenstrahl-Detektoreinrichtung (12) einen Bildverstärker und eine Fernsehkamera (14) enthält.

5. Verfahren zum Kompensieren der Streuschaltung bei einem Röntgenstrahl-Abbildungssystem gemäß Anspruch 1, mit den Schritten:
Positionieren einer Strahlungs-Abschirmeinrichtung (18) zwischen der Strahlungsquelle (10) und der Strahlungs-Detektoreinrichtung (12), dadurch gekennzeichnet, daß die von der Strahlungs-Detektoreinrichtung (12) empfangene Strahlung an den abgeschirmten Bereichen gemessen wird,
daß die Streustrahlung für alle Detektoreinrichtungen (12) von der gemessenen Strahlung an den abgeschirmten Bereichen interpoliert (22) wird,
daß die von der Strahlungs-Detektoreinrichtung (12) empfangene Strahlung an allen nicht abgeschirmten Bereichen gemessen wird, und
daß die interpolierte Streustrahlung von der gemessenen Röntgenstrahlung abgezogen (24) wird.

6. Verfahren nach Anspruch 5, wobei die Schritte der Strahlungsmessung gleichzeitig ablaufen.

7. Verfahren nach Anspruch 5 mit dem Schritt des Entfernens der Strahlungsabschirmung (18) und Messens der empfangenen Strahlung an allen Bereichen.

**Patensprüche fur die Vertragsstaaten: DE, NL**

1. Röntgenstrahl-Abbildungssystem mit:
einer Röntgenstrahlenquelle (10),
einer Röntgenstrahl-Detektoreinrichtung (12), die so angeordnet ist, daß sie von der Quelle Röntgenstrahlung empfängt und erfaßt und ein die Strahlung anzeigendes elektrisches Signal erzeugt,
eine Strahlungs-Abschirmeinrichtung (18), die zwischen der Quelle (10) und der Detektoreinrichtung (12) angeordnet ist und verhindert, daß Strahlung direkt zu abgeschirmten Bereichen der Detektoreinrichtung gelangt, gekennzeichnet durch eine Interpoliereinrichtung (22) zum Empfang elektrischer Signale von der Detektoreinrichtung (12), die von den abgeschirmten Bereichen der Detektoreinrichtung (12) empfangene Streuschaltung anzeigt und daraus ein Streustrahlungs-Signal für alle Detektoreinrichtungen (12) interpoliert, und eine Einrichtung (24) zum Subtrahieren des interpolierten Streustrahlungs-Signals von dem von der Detektoreinrichtung (12) erzeugten elektrischen Signal.

2. Röntgenstrahl-Abbildungssystem nach Anspruch 1, wobei die Abschirmeinrichtung (18) mehrere strahlungsundurchlässige Körper aufweist, die zwischen der Quelle (10) und der Detektoreinrichtung (12) angeordnet und räumlich verteilt sind, um die Interpolation eines Streustrahlungs-Signals für die gesamte Fläche der Detektoreinrichtung (12) zu erleichtern.

3. Röntgenstrahl-Abbildungssystem nach Anspruch 1 oder 2, wobei die Abschirmeinrichtung (18) einen Strahlkollimator (30) aufweist, um die Geometrie eines von der Detektoreinrichtung (12) direkt von der Quelle (10) empfangenen Röntgenstrahls zu begrenzen.

4. Röntgenstrahl-Abbildungssystem nach einem der vorstehenden Ansprüche, wobei die Röntgenstrahl-Detektoreinrichtung (12) einen Bildverstärker und eine Fernsehkamera (14) enthält.

**Revendications pour les etats Contractants: FR, GB**

1. Système de formation d'images radiographiques comprenant:

une source (10) de rayons X,

dys moyens détecteurs (12) de rayons X placés de façon à recevoir et détecter un rayonnement X provenant de la source et à générer un signal électrique qui en est représentatif,

des moyens de blindage (18) contre le rayonnement placés entre la source (10) et les moyens détecteurs (12) pour empêcher un rayonnement d'atteindre directement des zones protégées des moyens détecteurs, caractérisé en ce qu'il comporte des moyens (22) d'interpolation destinés à recevoir des signaux électriques provenant des moyens détecteurs (12), représentatifs du rayonnement diffusé reçu par les zones protégées des moyens détecteurs (12) et pour en interpoler un signal de rayonnement diffusé pour tous les moyens détecteurs (12), et

des moyens (24) destinés à soustraire le signal de rayonnement diffusé interpolé du signal électrique généré par les moyens détecteurs (12).

2. Système de formation d'images radiographiques selon la revendication 1, dans lequel les moyens (18) de blindage comprennent plusieurs corps opaques au rayonnement placés entre la source (10) et les moyens détecteurs (12) et répartis spatialement de façon à faciliter une interpolation d'un signal de rayonnement diffusé pour la totalité de l'étendue des moyens détecteurs (12).

3. Système de formation d'images radiographiques selon la revendication 1 ou la revendication 2, dans lequel les moyens de blindage (18) comprennent un colimateur (30) de faisceau destiné à limiter la géométrie du faisceau de rayons X reçu par les moyens détecteurs (12) directement de la source (10).

4. Système de formation d'images radiographiques selon l'une quelconque des revendications précédentes, dans lequel les moyens détecteurs (12) de rayons X comprennent un amplificateur de brilliance et une caméra (14) de télévision.

5. Procédé pour compenser le rayonnement diffusé dans un système de formation d'images radiographiques selon la revendication 1, comprenant les étapes qui consistent.

à placer des moyens (18) de blindage contre le rayonnement entre la source (10) de rayonnement et les moyens détecteurs (12) de rayonnement, caractérisé en ce qu'il consiste à mesurer le rayonnement reçu par les moyens détecteurs (12) de rayonnement dans les zones protégées,

à interpoler (22) le rayonnement diffusé pour la totalité des moyens détecteurs (12) à partir du rayonnement mesuré dans les zones protégées,

à mesurer le rayonnement reçu par les moyens détecteurs (12) dans toutes les zones non protégées, et

à soustraire (24) le rayonnement diffusé interpolé du rayonnement X mesuré.

6. Procédé selon la revendication 5, dans lequel les étapes de mesure du rayonnement sont simultanées.

7. Procédé selon la revendication 5, comprenant l'étape qui consiste à retirer le blindage (18) de protection contre les rayonnements et à mesurer le rayonnement reçu dans toutes les zones.

**Revendications pour les etats Contractants: DE, NL**

1. Système de formation d'images radiographiques comprenant:

une source (10) de rayons X,

des moyens détecteurs (12) de rayons X placés de façon à recevoir et détecter un rayonnement X provenant de la source et à générer un signal électrique qui en est représentatif,

des moyens de blindage (18) contre le rayonnement placés entre la source (10) et les moyens détecteurs (12) pour empêcher un rayonnement d'atteindre directement des zones protégées des moyens détecteurs, caractérisé en ce qu'il comporte des moyens (22) d'interpolation destinés à recevoir des signaux électriques provenant des moyens détecteurs (12), représentatifs du rayonnement diffusé reçu par les zones protégées des moyens détecteurs (12) et pour en interpoler un signal de rayonnement diffusé pour tous les moyens détecteurs (12), et

des moyens (24) destinés à soustraire le signal de rayonnement diffusé interpolé du signal électrique généré par les moyens détecteurs (12).

2. Système de formation d'images radiographiques selon la revendication 1, dans lequel les moyens (18) de blindage comprennent plusieurs corps opaques au rayonnement placés entre la source (10) et les moyens détecteurs (12) et répartis spatialement de façon à faciliter une interpolation d'un signal de rayonnement diffusé pour la totalité de l'étendue des moyens détecteurs (12).

3. Système de formation d'images radiographiques selon la revendication 1 ou la revendication 2, dans lequel les moyens de blindage (18) comprennent un colimateur (30) de faisceau destiné à limiter la géométrie du faisceau de rayons X reçu par les moyens détecteurs (12) directement de la source (10).

4. Système de formation d'images radiographiques selon l'une quelconque des revendications précédentes, dans lequel les moyens détecteurs (12) de rayons X comprennent un amplificateur de brilliance et une caméra (14) de télévision.

FIG.—1

FIG.—2

EP 0 105 618 B1